# EUROPEAN PATENT APPLICATION

(11) **EP 4 696 374 A1**
(43) Date of publication of application: **18.02.2026**
(21) Application number: 24825174.6
(22) Date of filing: 12.06.2024
(51) Int. Cl.: A61N 5/10

(54) **RADIOTHERAPY SYSTEM FOR ATRIAL FIBRILLATION**

(30) Priority: 21.06.2023 CN 202310744813
(71) Applicant: Beijing Baheal Guoxin Medical Technology, Beijing 102300 (CN)
(72) Inventor: XU, Huijun, Beijing 100166 (CN); LUO, Hangyu, Beijing 100164 (CN)
(74) Representative: E. Blum & Co. AG
(86) International application number: PCT/CN2024/098652
(87) International publication number: WO 2024/260278

(57) **Abstract**

The present disclosure provides a radiotherapy system for atrial fibrillation, including: a chamber; a support mounted in the chamber; a placement part located in the chamber and fixedly connected to the bottom of the chamber, wherein the placement part is used for adjusting the patient's posture; a detection part configured to detect an atrial fibrillation target area, wherein the detection part includes a ring gantry, a plurality of X-ray tubes, and a plurality of X-ray detectors, the ring gantry is rotatably mounted at the top of the support around the axis of the ring gantry, at least some of the plurality of X-ray detectors are arranged in an arc shape on the inner side of the ring gantry, and at least some of the plurality of X-ray tubes are arranged in an arc shape on the inner side of the ring gantry; and a treatment part configured to perform radiotherapy on the atrial fibrillation target area, wherein a treatment end of the treatment part is fixedly mounted at the end of the ring gantry away from the placement part, and a driving end of the treatment part is fixedly connected to the support. The problem that a complete image of the heart of a patient cannot be acquired because a traditional treatment device cannot move to track an irradiation target area and cannot achieve volume imaging is solved.

## Description

### Cross-Reference to Related Applications

The present disclosure is based on and claims priority to CN Application No. 202310744813.0 filed on June 21, 2023, the disclosures of which are hereby incorporated by reference herein in their entireties.

### Technical Field

The present disclosure belongs to the technical field of medical radiation therapy devices, and particularly relates to a radiotherapy system for atrial fibrillation.

### Background

Atrial fibrillation (AF) is a common arrhythmia. It refers to the loss of regular and ordered atrial electrical activity, replaced by rapid and disorderly fibrillation waves, and is a serious atrial electrical activity disorder. It often characterized as irregular and rapid heart rate, results from the loss of normal rhythm in the atria. Atrial fibrillation not only affects patients' quality of life, but can also lead to serious complications such as thromboembolism and heart failure, with the most serious complication being stroke.

Radiation therapy is one of the three major treatments for tumors. External beam radiotherapy involves radiation beams incident on the body from outside, which is a non-invasive procedure. The external beam devices include linear accelerators (LINAC), cobalt-60 radioactive source, radiation source of the proton, heavy ion, etc. Radiation beams are directed at the tumor region from coplanar or non-coplanar directions, and superposing and focusing in the tumor area, resulting in a high dose accumulation in the tumor region while relatively lower doses in normal tissues. Since tumors are systemic, a larger irradiation space with fewer blind spots is preferable. A larger irradiation space allows for better beam arrangement, better avoidance and protection of normal organs.

Among the commonly available treatment devices on the market, one type of conventional accelerator uses a C-arm structure, where the patient rotates 360 degrees, but the C-arm support cannot move dynamically to track the irradiation target area. Another type uses an X-ray tube fixed to the ceiling and a flat panel fixed to the floor, which only captures a small range of images at a 45-degree angle, making it impossible to perform volume imaging and thus unable to obtain a complete image of the patient's heart. To address the above issues, the following solution is proposed.

### Summary

To solve the above problem, the present disclosure provides a radiotherapy system for atrial fibrillation, including:
a chamber;
a support mounted in the chamber;
a placement part located in the chamber and fixedly connected to a bottom of the chamber for adjusting a patient's posture;
a detection part configured to detect an atrial fibrillation target area, the detection part includes a ring gantry, a plurality of X-ray tubes, and a plurality of X-ray detectors, the ring gantry is rotatably mounted at the top of the support around its own axis, at least some of the plurality of X-ray detectors are arranged in an arc shape on the inner side of the ring gantry, and at least some of the plurality of X-ray tubes are arranged in an arc shape on the inner side of the ring gantry; and
a treatment part configured to perform radiotherapy on the atrial fibrillation target area, wherein a treatment end of the treatment part is fixedly mounted at the end of the ring gantry away from the placement part, and a driving end of the treatment part is fixedly connected to the support.

In some embodiments, the radiotherapy system for atrial fibrillation further includes:
a tracking part configured to acquire a movement of a patient's body surface, wherein the tracking part is located above the placement part, and a detection end of the tracking part faces to a placement end of the placement part, and the tracking part is fixedly connected to the top of the chamber.

In some embodiments, the treatment part includes a robotic arm and a radiation source, wherein the robotic arm is mounted on the ring gantry, and the radiation source is mounted on an output end of the robotic arm, and the ring gantry is configured to adjust an irradiation angle of the radiation source in a circumferential direction by rotating, and the robotic arm is configured to follow the movement of the patient's body surface to adjust an irradiation position of the radiation source.

In some embodiments, the radiotherapy system for atrial fibrillation further includes a driving component, the driving component includes a rotating ring, a motor, a bearing, and a drive roller, the rotating ring is fixed to the end of the ring gantry away from the placement part, and the rotating ring is connected to the support via the bearing, and the motor is mounted on the side of the support, and the drive roller is mounted on an output end of the motor and used for driving the rotating ring to rotate.

In some embodiments, the treatment part includes a robotic arm, a radiation source, and a collimator, wherein the robotic arm is mounted on an end of the rotating ring adjacent to the ring gantry, and the radiation source is mounted on an output end of the robotic arm, and the collimator is mounted on an output end of the radiation source.

In some embodiments, the radiotherapy system is configured to have at least one of:
the radiation source is any one of an electron linear accelerator, a cobalt source, a proton accelerator, or a heavy ion accelerator; and
the collimator is any one of a cone collimator or a multi-leaf collimator; and
the X-ray tube is a cold cathode X-ray tube.

In some embodiments, the placement part includes a multi-degree-of-freedom robotic arm and a treatment couch, wherein the multi-degree-of-freedom robotic arm is mounted on the bottom of the chamber, and the treatment couch is mounted on an output end of the multi-degree-of-freedom robotic arm, and an initial state of the treatment couch is horizontal.

Optionally, the multi-degree-of-freedom robotic arm is a six-axis robotic arm.

In some embodiments, the radiotherapy system for atrial fibrillation further includes: a tracking part configured to acquire a movement of patient's body surface, wherein the tracking part includes an optical measurement camera and a marking part, and the optical measurement camera is fixed to the top surface in the chamber, and the marking part is configured to be mounted on the patient lying on the treatment couch.

In some embodiments, a control console is disposed on one side of the support, and the placement part, the tracking part, the treatment part, and the detection part are all electrically connected to the control console.

In some embodiments, the X-ray tubes and the X-ray detectors within a same cross-section of the ring gantry form a detection group, and at least one detection group is disposed in the ring gantry; Within the same cross-section of the ring gantry, there is a gap between one end of the arc-shaped X-ray detectors and one end of the arc-shaped X-ray tubes for the treatment part to perform treatment.

In some embodiments, there are a plurality of detection groups, and the adjacent detection groups are staggered around a circumferential direction of the ring gantry to form a detection array, and the gaps of the plurality of detection groups are all located on a side of the ring gantry close to the treatment part.

In some embodiments, there are a plurality of detection groups, and the plurality of detection groups are mutually independent, and each of the plurality of detection groups can be selectively connecting or disconnecting.

In some embodiments, each detection group is equipped with an anode copper bar connected to an anode of the X-ray tubes and an anode of the X-ray detectors, wherein the anode copper bar is located behind the detection group and embedded in a non-gap segment of the ring gantry, and each detection group is equipped with an anode high-voltage socket, and the anode copper bar is fixedly connected to the anode high-voltage socket, and the anode high-voltage sockets of the plurality of detection groups are arranged along an axial direction of the ring gantry, and an insulating plate is movably inserted into the anode high-voltage socket, the insulating plate is configured to cut off or connect a current between the anode high-voltage socket and an external source by moving.

In some embodiments, the anode high-voltage socket includes an upper socket and a lower socket fixedly connected via an eccentric shaft, wherein the upper socket is fixedly connected to the anode copper bar, and there is a channel for the insulating plate to move between the upper socket and the lower socket, and a combination body is rotatably mounted at the outer end of the eccentric shaft, the combination body including a conductive portion and an insulating portion. The insulating plate and the combination body share a space of the channel, and a side of the insulating portion has a beveled surface for matching with the insulating plate.

In some embodiments, the arc-shaped X-ray tubes and the arc-shaped X-ray detectors are arranged spaced apart along an axial direction of the ring gantry. The X-ray tubes are arranged along an entire circumference within the same cross-section of the ring gantry, with a gap for the treatment part to perform treatment. The X-ray detectors are arranged along the entire circumference within the same cross-section of the ring gantry, with the gap for the treatment part to perform treatment.

### Brief Description of the Drawings

The drawings described herein are provided for further understanding of the present disclosure and constitute a part of this application. The schematic embodiments of the present disclosure and their descriptions are used to explain the present disclosure and do not constitute an undue limitation of the present disclosure. In the drawings:
FIG. 1 is a schematic structural diagram of some embodiments of the radiotherapy system for atrial fibrillation of the present disclosure;
FIG. 2 is a schematic diagram of the placement part, the tracking part, and the treatment part;
FIG. 3 is a schematic diagram of the relative position relationship between the detection part and the radiation source;
FIG. 4 is a schematic diagram of the detection array;
FIG. 5 is a schematic sectional view of the detection array;
FIG. 6 is a schematic diagram of multiple pairs of independently installed detection groups;
FIG. 7 is a structural schematic diagram of a detection group and an anode high-voltage socket;
FIG. 8 is a perspective view of the anode high-voltage socket;
FIG. 9 is a schematic diagram showing the change of the anode high-voltage socket from cutting off current to conducting current.

The reference numerals are represented as follows:
1. Support; 2. Chamber; 3. Placement part; 4. Detection part; 5. Tracking part; 6. Treatment part; 7. Ring gantry; 8. X-ray tube; 9. X-ray detector; 10. Gap; 11. Detection group; 12. Rotating ring; 13. Motor; 14. Bearing; 15. Drive roller; 16. Robotic arm; 17. Radiation source; 18. Collimator; 19. Multi-degree-of-freedom robotic arm; 20. Treatment couch; 21. Optical measurement camera; 23. Control console; 24. Detection array; 25. Lower socket; 26. Upper socket; 27. Combination body; 271. Conductive portion; 272. Insulating portion; 273. Eccentric shaft; 28. Insulating plate; 29. Anode copper bar.

### Detailed Description

The present disclosure is described in detail below. The following paragraphs define different aspects of the embodiments in more detail. The aspects defined as such may be combined with any other one or more aspects unless explicitly indicated as non-combinable. In particular, any feature considered preferred or advantageous may be combined with one or more other features considered preferred or advantageous.

The terms "first," "second," etc., appearing in the present disclosure are merely for convenience of description to distinguish components with the same name and do not denote sequence or primary-secondary relationships.

In the description of the present disclosure, the orientation or position relation indicated by "up", "down", " top", " bottom", " front", "back", "in" and "outside", etc., is based on the orientation or position relation shown in the drawings, and is merely for describing the present disclosure. These location nouns do not indicate or imply that the indicated apparatus must have a particular orientation or be constructed and operated in a particular orientation, and thus cannot be interpreted as limitations to the protection scope of the present disclosure.

To address the problems in the prior art, the objective of the present disclosure is to provide a radiotherapy system for atrial fibrillation, which solves the problems that a complete image of the heart of a patient cannot be acquired because a traditional treatment device cannot move to track an irradiation target area and cannot achieve volume imaging.

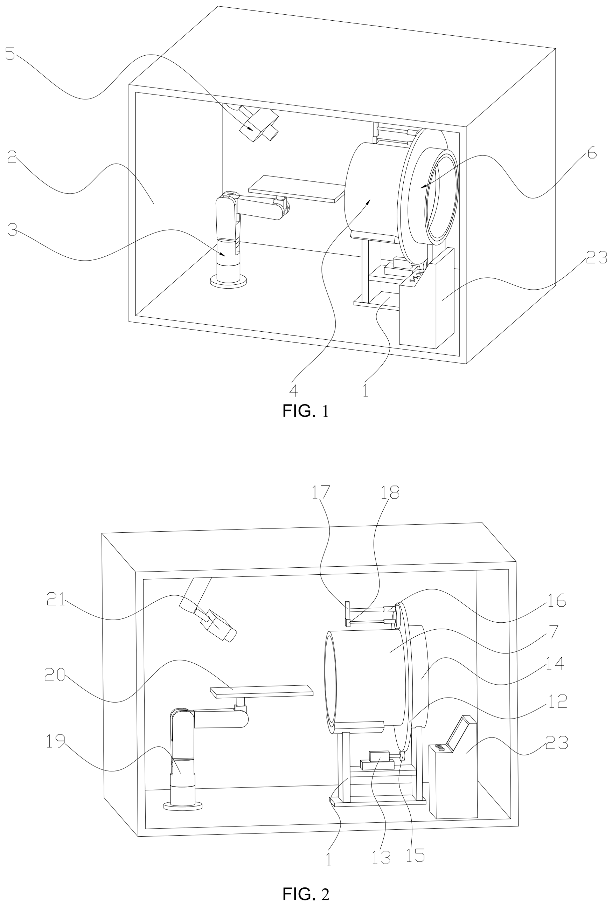

As shown in FIGS. 1-5, an embodiment of the present disclosure provides a radiotherapy system for atrial fibrillation based on a robotic arm, including a support 1, a chamber 2 for mounting the support 1, a placement part 3 for adjusting a patient's posture, a detection part 4 for detecting an atrial fibrillation target area, a tracking part 5 for acquiring a movement of a patient's body surface, and a treatment part 6 for performing radiotherapy on the atrial fibrillation target area, and a control console 23 is disposed on one side of the support 1. The placement part 3, the tracking part 5, the treatment part 6, and the detection part 4 are all electrically connected to the control console 23.

The support 1 is mounted in the chamber 2. The placement part 3 is located in the chamber 2. The placement part 3 includes a multi-degree-of-freedom robotic arm 19 and a treatment couch 20. The multi-degree-of-freedom robotic arm 19 is a six-axis robotic arm. The multi-degree-of-freedom robotic arm 19 is mounted on the bottom of the chamber 2. The treatment couch 20 is mounted on an output end of the multi-degree-of-freedom robotic arm 19. The treatment couch 20 is made of a material such as carbon fiber that is penetrable by a radiation source 17. An initial state of the treatment couch 20 is horizontal.

The multi-degree-of-freedom robotic arm 19 is used to support the treatment couch 20 to support the patient, and the multi-degree-of-freedom robotic arm 19 enables multi-angle adjustment of the patient's position, facilitating subsequent detection without requiring the patient to move themselves, making it more convenient and less strenuous. Simultaneously, the treatment couch 20 is made of a material such as carbon fiber that is penetrable by the radiation source 17, facilitating the penetration of the radiation source 17 and treatment of the patient.

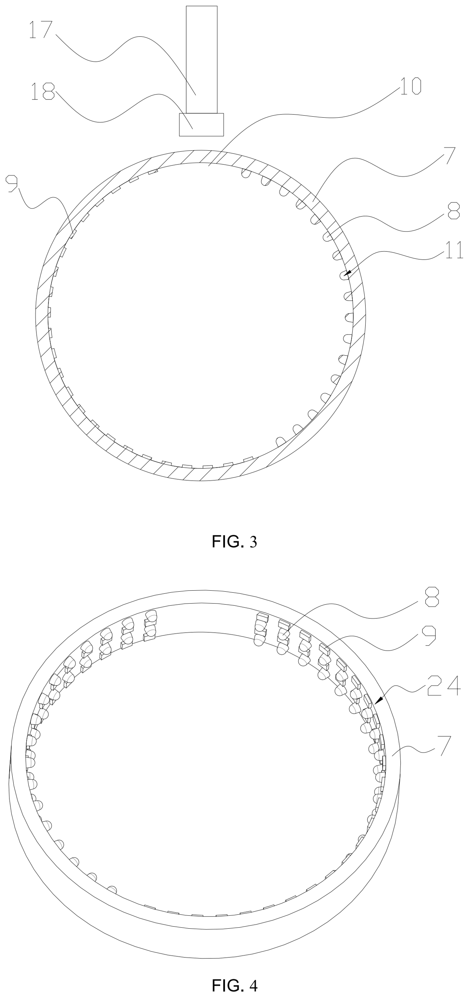

As shown in FIG. 3, the detection part 4 includes a ring gantry 7, a plurality of X-ray tubes 8, and a plurality of X-ray detectors 9. The X-ray tubes 8 are cold cathode X-ray tubes. The ring gantry 7 is rotatably mounted at the top of the support 1 around its own axis. The axis of the ring gantry 7 is horizontally disposed. At least some of the plurality of X-ray detectors 9 are arranged in an arc shape on the inner side of the ring gantry 7. At least some of the plurality of X-ray tubes 8 are arranged in an arc shape on the inner side of the ring gantry7.

For example, as shown in FIGS. 3 to 6, the arc-shaped X-ray detectors 9 are located within the same cross-section of the ring gantry 7. The X-ray tubes 8 are arranged in an arc shape, and the arc-shaped X-ray tubes 8 and the arc-shaped X-ray detectors 9 are located within the same cross-section. One end of the arc-shaped X-ray detectors 9 and one end of the arc-shaped X-ray tubes 8 define a gap 10 for the treatment part 6 to perform treatment. The X-ray tubes 8 and X-ray detectors 9 within the same cross-section form a detection group 11. At least one detection group 11 is disposed in the ring gantry 7.

This embodiment positions the treatment part 6 on the ring gantry 7, allowing it to rotate with the ring gantry 7 to irradiate the patient from different angular positions. Moreover, the use of arc-shaped X-ray tubes 8 and X-ray detectors 9 in cooperation allows scanning the patient and constructing a complete three-dimensional image of the patient without rotating the patient or the detection group 11. Not rotating the patient ensures patient comfort, and a stationary detection group 11 improves safety during detection. The complete three-dimensional image facilitates determining the target area for subsequent precise radiotherapy. Therefore, the embodiment of the present disclosure does not require high-speed rotation of the detection group 11 to obtain a three-dimensional image; rotation of the ring gantry 7 is solely for treatment, whereas prior art static CT requires high-speed rotation of the detection group 11 to obtain a three-dimensional image.

In some embodiments, the placement part 3 includes a treatment couch, the treatment part 6 includes an irradiation system, and the detection part 4 includes an imaging system.

In some embodiments, the radiotherapy system for atrial fibrillation of the present disclosure further includes: a tracking part 5 configured to acquire a movement of a patient's body surface. The tracking part 5 is located above the placement part 3. A detection end of the tracking part 5 faces to a placement end of the placement part 3, and the tracking part 5 is fixedly connected to the top of the chamber 2.

As shown in FIG. 2, the tracking part 5 is located above the treatment couch 20. The tracking part 5 includes an optical measurement camera 21 and a marking part. The optical measurement camera 21 is fixed to the top surface in the chamber 2. The marking part is configured to be mounted on the patient lying on the treatment couch 20. The optical measurement camera 21 records the marking part fixed on the human body in real time to establish a respiratory dynamic model of the patient, ensuring the accuracy of subsequent radiotherapy.

In some embodiments, the treatment part 6 includes a robotic arm 16 and a radiation source 17. The robotic arm 16 is mounted on the ring gantry 7. The radiation source 17 is mounted on an output end of the robotic arm 16. The ring gantry 7 is configured to adjust an irradiation angle of the radiation source 17 in a circumferential direction by rotating. The robotic arm 16 is configured to adjust an irradiation position of the radiation source 17 by moving in response to patient surface motion.

Based on the detection of the movement of the patient's body surface by the tracking part 5, this embodiment allows adjustment of the irradiation direction of the treatment part 6 through rotation of the ring gantry 7 and adjustment of the irradiation position of the radiation source 17 through movement of the robotic arm 16, thereby enabling precise irradiation of the area requiring treatment on the patient.

As shown in FIG. 2, the radiotherapy system for atrial fibrillation of the present disclosure further includes a driving component. The driving component includes a rotating ring 12, a motor 13, a bearing 14, and a drive roller 15. The rotating ring 12 is fixed to an end of the ring gantry 7 away from the placement part 3. The rotating ring 12 is connected to the support 1 via the bearing 14 for installation. The motor 13 is mounted on the side of the support 1. The drive roller 15 is mounted on the output end of the motor 13 and used for driving the rotating ring 12 to rotate.

In this embodiment, the motor 13 can drive the rotating ring 12 to rotate, thereby driving the ring gantry 7 to rotate, and allows precise control of the rotational position.

In some embodiments, the treatment part 6 includes the robotic arm 16, a radiation source 17, and a collimator 18. The robotic arm 16 is mounted on the rotating ring 12 adjacent to the ring gantry 7. The radiation source 17 is mounted on the output end of the robotic arm 16. The collimator 18 is mounted on the output end of the radiation source 17. The radiation source 17 is any one of an electron linear accelerator, a cobalt source, a proton accelerator, or a heavy ion accelerator. The collimator 18 is any one of a cone collimator or a multi-leaf collimator.

After the three-dimensional image is established, the radiation source 17 irradiates the target area. The rotating ring 12 facilitates the radiation source 17 to rotate 360 degrees around the patient for precise treatment of the target area. Different radiation sources 17 and replaceable collimators 18 are suitable for different needs.

In some embodiments, the X-ray tubes 8 and the X-ray detectors 9 within the same cross-section of the ring gantry 7 form a detection group 11. At least one detection group 11 is disposed in the ring gantry 7. Within the same cross-section of the ring gantry 7, one end of the arc-shaped X-ray detectors 9 and one end of the arc-shaped X-ray tubes 8 define the gap 10 for the treatment part 6 to perform treatment. Radiation emitted by an X-ray tube 8 can be received and imaged by the X-ray detector 9 within the same detection group 11. This embodiment achieves imaging using a detection group 11 within the same cross-section.

As shown in FIG. 3, the gap on the detection group 11 faces to the radiation source 17, facilitating penetration of radiation source 17. The rotating ring 12 is fixedly connected to the ring gantry 7, ensuring that when the radiation source 17 rotates, it drives the detection group 11 to rotate synchronously, thereby maintaining alignment between the gap and the radiation source 17.

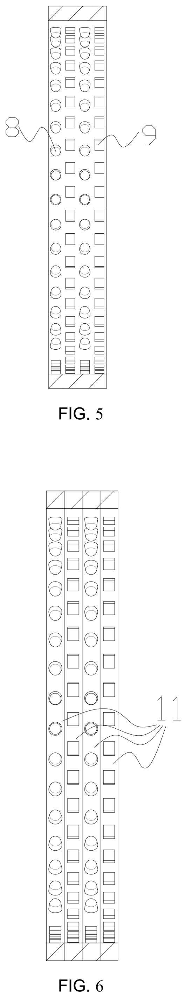

As shown in FIGS. 4 and 5, there are a plurality of detection groups 11, and adjacent detection groups 11 are staggered around the circumference of the ring gantry 7 to form a detection array 24. The gaps 10 of the plurality of detection groups 11 are all located on the side of the ring gantry 7 close to the treatment part 6.

The detection array 24 with a plurality of detection groups 11 have higher detection efficiency, while the staggered arrangement manner of adjacent detection groups 11 within the array can improve detection accuracy.

In some embodiments, there are a plurality of detection groups 11. The plurality of detection groups 11 are mutually independent, and each detection group 11 can be selectively connecting or disconnecting. This embodiment allows imaging to continue via other detection groups 11 if some detection groups 11 develop faulty pixels or fail, meeting usage demands and improving overall lifespan. Moreover, activating a plurality of detection groups 11 simultaneously enables a larger imaging range, for example, the number of detection groups 11 activated can be judged based on the size of the tumor region.

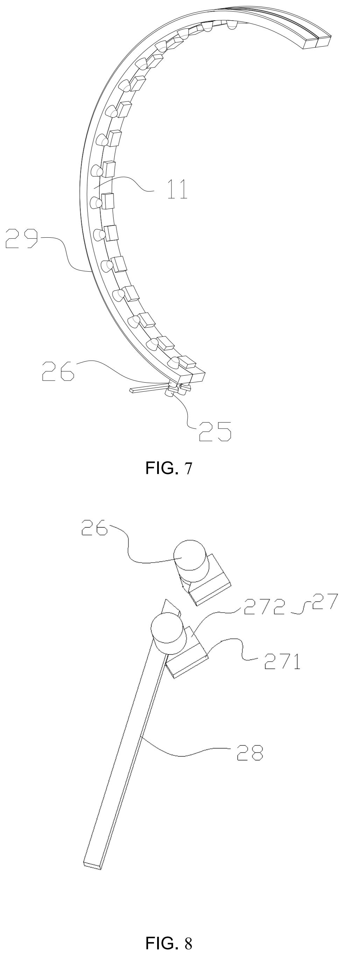

As shown in FIG. 6, a plurality of detection groups 11 are separate and independently installed on the inner side wall of the ring gantry 7. As shown in FIGS. 7 to 9, each detection group 11 is equipped with an anode copper bar 29 connected to the anodes of the cold cathode X-ray tubes 8 and the anodes of the X-ray detectors 9 within that group. The anode copper bar 29 is located behind the detection group 11 and embedded in a non-gap segment of the ring gantry 7. Corresponding to each detection group 11, an anode high-voltage socket is provided. The anode copper bar 29 is fixedly connected to the anode high-voltage socket. The anode high-voltage sockets of a plurality of detection groups 11 are arranged along the axial direction of the ring gantry 7. An insulating plate 28 is movably inserted within the anode high-voltage socket (or between sockets). The movement of the insulating plate 28 controls the cutting off or connecting of the current between the anode high-voltage socket and an external source. It should be noted that the control movement of the insulating plate 28 can be achieved through existing technologies like drive motors or PLC control, which will not be elaborated here.

Optionally, the anode high-voltage socket includes an upper socket 26 and a lower socket 25 fixedly connected together via an eccentric shaft 273. The upper socket 26 is fixedly connected to the anode copper bar 29. A channel for the insulating plate 28 to move is provided between the upper socket 26 and the lower socket 25. A combination body 27, including a conductive portion 271 and an insulating portion 272, is rotatably disposed at an outer end of the eccentric shaft 273. The insulating plate 28 and the combination body 27 share the space within the channel. The combination body 27 has a beveled surface on the side of the insulating portion 272 for matching with the insulating plate 28, which facilitates the insulating plate 28 in pushing the combination body 27 to rotate more effectively.

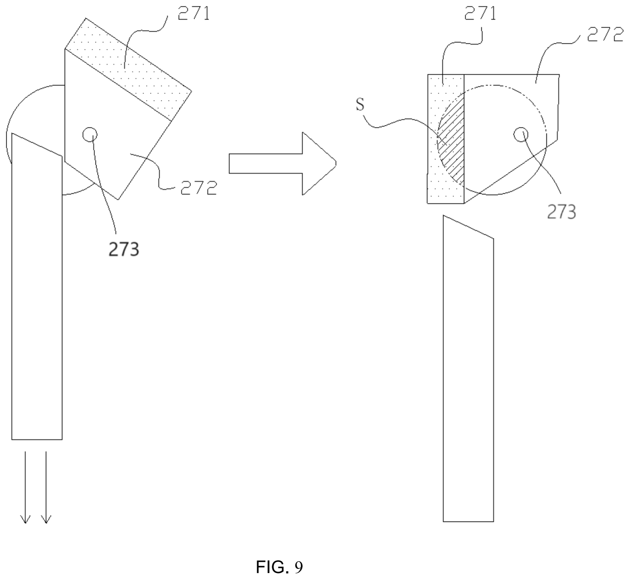

When the insulating plate 28 is moved under operator control, it pushes the combination body 27 to rotate, causing the conductive portion 271 to rotate out from between the upper socket 26 and the lower socket 25. The insulating portion 272 then contacts the upper socket 26 and the lower socket 25, thereby cutting off the current connection of the anode high-voltage socket. Conversely, when the insulating plate 28 is withdrawn, the combination body 27, under its own restoring force (e.g., from a torsion spring), naturally returns the rotated-out conductive portion 271 to a position contacting both the upper socket 26 and the lower socket 25, i.e., the process shown in FIG. 9. Area S represents the contact region between the upper sockets and lower sockets, completing current conduction. By controlling the movement of the insulating plate 28, the required number of detection groups 11 can be selected to work, providing high controllability. Alternatively, selecting a portion of the detection groups 11 to activate first ensures that when detection accuracy decreases over time due to aging or performance degradation, adding more detection groups 11 can maintain a certain level of detection accuracy.

In other embodiments, the arc-shaped X-ray tubes 8 and the arc-shaped X-ray detectors 9 are arranged spaced apart along the axial direction of the ring gantry 7. Within the same cross-section of the ring gantry 7, X-ray tubes 8 are arranged along the entire circumference, with the gap 10 defined for the treatment part 6 to perform treatment. Within the same cross-section of the ring gantry 7, X-ray detectors 9 are arranged along the entire circumference, with the gap 10 defined for the treatment part 6 to perform treatment. An X-ray tube 8 can emit rays towards the X-ray detectors 9 on its left, right, or both sides; both sides can be irradiated, increasing the imaging range. With this distributed arrangement of X-ray tubes 8, different regions of the patient can be imaged without rotating the ring gantry 7.

During use, for a ring of X-ray tubes 8, multiple continuously arranged X-ray tubes 8 can be selected for imaging. By selecting X-ray tubes 8 in different angular regions, imaging can be performed from different angular directions.

To solve the above problems, the present disclosure adopts the following technical solution.

A radiotherapy system for atrial fibrillation based on a robotic arm, includes a support and a chamber for mounting the support. It further includes a placement part for adjusting a patient's posture, a detection part for detecting an atrial fibrillation target area, a tracking part for acquiring a movement of a patient's body surface, and a treatment part for performing radiotherapy on the atrial fibrillation target area;
the support is mounted in the chamber;
the placement part is located in the chamber, and the placement part is fixedly connected to the bottom of the chamber;
the tracking part is located above the placement part, a detection end of the tracking part faces to a placement end of the placement part, and the tracking part is fixedly connected to the top of the chamber;
the detection part includes a ring gantry, X-ray tubes, and X-ray detectors. The ring gantry is rotatably mounted at the top of the support. A plurality of X-ray detectors are arranged in an arc shape on the inner side of the ring gantry. The arc-shaped X-ray detectors are located within the same cross-section of the ring gantry. The X-ray tubes are arranged in an arc, and the arc-shaped X-ray tubes and the arc-shaped X-ray detectors are located within the same cross-section. One end of the arc-shaped X-ray detectors and one end of the arc-shaped X-ray tubes define the gap for the treatment part to perform treatment. The X-ray tubes and X-ray detectors within the same cross-section form a detection group. At least one detection group is disposed in the ring gantry;
a treatment end of the treatment part is fixedly mounted at an end of the ring gantry away from the placement part, and a driving end of the treatment part is fixedly connected to the support.

In some embodiments, the treatment part includes a rotating ring, a motor, a bearing, a drive roller, a robotic arm, a radiation source, and a collimator. The rotating ring is fixed to an end of the ring gantry away from the placement part. The rotating ring is connected to the support via the bearing. The robotic arm is mounted on the rotating ring adjacent to the ring gantry. The radiation source is mounted on an output end of the robotic arm. The collimator is mounted on an output end of the radiation source. The motor is mounted on a side of the support. The drive roller is mounted on an output end of the motor and configured to drive the rotating ring to rotate.

In some embodiments, the placement part includes a multi-degree-of-freedom robotic arm and a treatment couch. The multi-degree-of-freedom robotic arm is mounted on the bottom of the chamber. The treatment couch is mounted on an output end of the multi-degree-of-freedom robotic arm, and an initial state of the treatment couch is horizontal.

In some embodiments, the tracking part includes an optical measurement camera and a marking part. The optical measurement camera is fixed to an inner top surface of the chamber. The marking part is attached to a patient lying on the treatment couch.

In some embodiments, the X-ray tube is a cold cathode X-ray tube.

In some embodiments, the radiation source is any one of an electron linear accelerator, a cobalt source, a proton accelerator, or a heavy ion accelerator.

In some embodiments, the collimator is any one of a cone collimator or a multi-leaf collimator.

In some embodiments, a control console is disposed on one side of the support. The placement part, the tracking part, the treatment part, and the detection part are all electrically connected to the control console.

In some embodiments, the multi-degree-of-freedom robotic arm is a six-axis robotic arm.

In some embodiments, there are a plurality of detection groups, and adjacent detection group are staggered from each other to form a detection array. The gaps of the plurality of detection groups are all located on a side of the ring gantry close to the treatment part.

Advantages of the embodiments of the present disclosure compared to the related art:
(1) This solution utilizes arc-shaped X-ray tubes and X-ray detectors in cooperation, allowing scanning of the patient and construction of a complete three-dimensional image without rotating the patient or the detection group. Not rotating the patient ensures comfort, and a stationary detection group improves safety during detection. The complete three-dimensional image facilitates target area determination and enables precise subsequent radiotherapy.
(2) The optical measurement camera records markers fixed to the human body in real-time to establish a respiratory dynamic model of the patient, ensuring the accuracy of subsequent radiotherapy.
(3) After the three-dimensional image is established, the radiation source irradiates the target area. The rotating ring facilitates the radiation source rotating 360 degrees around the patient for precise treatment. Replaceable collimators suit different requirements.
(4) Using a multi-degree-of-freedom robotic arm to support the treatment couch allows multi-angle adjustment of the patient's position, facilitating subsequent detection without requiring the patient to move, making it more convenient and less strenuous. Simultaneously, the radiation-penetrable material (e.g., carbon fiber) of the treatment couch facilitates radiation penetration and patient treatment.
(5) The detection array composed of multiple detection group provides higher detection efficiency. The staggered arrangement manner of adjacent units within the array improves detection accuracy.
(6) The gap on the detection group faces to the radiation source, facilitating penetration. The fixed connection between the rotating ring and the ring gantry ensures the gap and radiation source remain aligned during rotation.
(7) Multiple independently installed detection group, with anode high-voltage sockets controlling current flow, allow selection of the required number of units, providing high controllability. When detection accuracy decreases over time due to aging or performance degradation, selectively adding units maintains accuracy.
(8) As of 2019, there were approximately 59.7 million global AF patients (including atrial flutter); among people of European descent over 55 years old, one in three has AF. According to epidemiological survey data from China published in The Lancet in April 2022, the crude prevalence of AF in China is 2.3%, positively correlated with age. Based on the Sixth National Population Census data, the age-standardized prevalence of AF in China is 1.6%, estimating 20 million patients. Therefore, there is a significant unmet clinical need for non-invasive AF treatment. Based on this, the present disclosure proposes, for the first time, a dedicated radiotherapy device for treating cardiovascular diseases, solving clinical pain points and possessing immense industrial value.

Although some specific embodiments of the present disclosure have been described in detail by way of example, those skilled in the art should understand that the above examples are for illustrative purposes only and are not intended to limit the scope of the present disclosure. Those skilled in the art should understand that modifications to the above embodiments or equivalent substitutions of some technical features may be made without departing from the scope and spirit of the present disclosure. The scope of the present disclosure is defined by the appended claims.

## Claims

1. A radiotherapy system for atrial fibrillation, comprising:
a chamber (2);
a support (1) mounted in the chamber (2);
a placement part (3) located in the chamber (2) and fixedly connected to a bottom of the chamber (2) for adjusting a patient's posture;
a detection part (4) configured to detect an atrial fibrillation target area, the detection part (4) comprises a ring gantry (7), a plurality of X-ray tubes (8), and a plurality of X-ray detectors (9), the ring gantry (7) is rotatably mounted at the top of the support (1) around its own axis, at least some of the plurality of X-ray detectors (9) are arranged in an arc shape on the inner side of the ring gantry (7), and at least some of the plurality of X-ray tubes (8) are arranged in an arc shape on the inner side of the ring gantry (7); and
a treatment part (6) configured to perform radiotherapy on the atrial fibrillation target area, wherein a treatment end of the treatment part (6) is fixedly mounted at the end of the ring gantry (7) away from the placement part (3), and a driving end of the treatment part (6) is fixedly connected to the support (1).

2. The radiotherapy system for atrial fibrillation according to claim 1, further comprising:
a tracking part (5) configured to acquire a movement of a patient's body surface, wherein the tracking part (5) is located above the placement part (3), and a detection end of the tracking part (5) faces to a placement end of the placement part (3), and the tracking part (5) is fixedly connected to the top of the chamber (2).

3. The radiotherapy system for atrial fibrillation according to claim 2, wherein the treatment part (6) comprises a robotic arm (16) and a radiation source (17), wherein the robotic arm (16) is mounted on the ring gantry (7), and the radiation source (17) is mounted on an output end of the robotic arm (16), and the ring gantry (7) is configured to adjust an irradiation angle of the radiation source (17) in a circumferential direction by rotating, and the robotic arm (16) is configured to follow the movement of the patient's body surface to adjust an irradiation position of the radiation source (17).

4. The radiotherapy system for atrial fibrillation according to any one of claims 1 to 3, further comprising:
a driving component, the driving component comprises a rotating ring (12), a motor (13), a bearing (14), and a drive roller (15), the rotating ring (12) is fixed to the end of the ring gantry (7) away from the placement part (3), and the rotating ring (12) is connected to the support (1) via the bearing (14), and the motor (13) is mounted on the side of the support (1), and the drive roller (15) is mounted on an output end of the motor (13) and used for driving the rotating ring (12) to rotate.

5. The radiotherapy system for atrial fibrillation according to claim 4, wherein the treatment part (6) comprises a robotic arm (16), a radiation source (17), and a collimator (18), wherein the robotic arm (16) is mounted on an end of the rotating ring (12) adjacent to the ring gantry (7), and the radiation source (17) is mounted on the output end of the robotic arm (16), and the collimator (18) is mounted on an output end of the radiation source (17).

6. The radiotherapy system for atrial fibrillation according to claim 5, wherein the radiotherapy system is configured to have at least one of:
the radiation source (17) is any one of an electron linear accelerator, a cobalt source, a proton accelerator, or a heavy ion accelerator; and
the collimator (18) is any one of a cone collimator or a multi-leaf collimator; and
the X-ray tube (8) is a cold cathode X-ray tube.

7. The radiotherapy system for atrial fibrillation according to any one of claims 1 to 6, wherein the placement part (3) comprises a multi-degree-of-freedom robotic arm (19) and a treatment couch (20), wherein the multi-degree-of-freedom robotic arm (19) is mounted on the bottom of the chamber (2), and the treatment couch (20) is mounted on an output end of the multi-degree-of-freedom robotic arm (19), and an initial state of the treatment couch (20) is horizontal; optionally,
the multi-degree-of-freedom robotic arm (19) is a six-axis robotic arm.

8. The radiotherapy system for atrial fibrillation according to claim 7, further comprising:
a tracking part (5) configured to acquire a movement of a patient's body surface, wherein the tracking part (5) comprises an optical measurement camera (21) and a marking part, and the optical measurement camera (21) is fixed to the top surface in the chamber (2), and the marking part is configured to be mounted on the patient lying on the treatment couch (20).

9. The radiotherapy system for atrial fibrillation according to any one of claims 1 to 8, wherein a control console (23) is disposed on one side of the support (1), and the placement part (3), the tracking part (5), the treatment part (6) and the detection part (4) are all electrically connected to the control console (23).

10. The radiotherapy system for atrial fibrillation according to any one of claims 1 to 9, wherein the X-ray tubes (8) and the X-ray detectors (9) within a same cross-section of the ring gantry (7) form a detection group (11), and at least one detection group (11) is disposed in the ring gantry (7); within the same cross-section of the ring gantry (7), there is a gap (10) between one end of the arc-shaped X-ray detectors (9) and one end of the arc-shaped X-ray tubes (8) for the treatment part (6) to perform treatment.

11. The radiotherapy system for atrial fibrillation according to claim 10, wherein there are a plurality of detection groups (11), and adjacent detection groups (11) are staggered around a circumferential direction of the ring gantry (7) to form a detection array (24), and the gaps (10) of the plurality of detection groups (11) are all located on a side of the ring gantry (7) close to the treatment part (6).

12. The radiotherapy system for atrial fibrillation according to claims 10 or 11, wherein there are a plurality of detection groups (11), and the plurality of detection groups (11) are mutually independent, and each of the plurality of detection groups (11) can be selectively connecting or disconnecting.

13. The radiotherapy system for atrial fibrillation according to any one of claims 10 to 12, wherein each detection group (11) is equipped with an anode copper bar (29) connected to an anode of the X-ray tubes (8) and an anode of the X-ray detectors (9), wherein the anode copper bar (29) is located behind the detection group (11) and embedded in a non-gap segment of the ring gantry (7), and each detection group (11) is equipped with an anode high-voltage socket, and the anode copper bar (29) is fixedly connected to the anode high-voltage socket, and the anode high-voltage sockets of the plurality of detection groups (11) are arranged along an axial direction of the ring gantry (7), and an insulating plate (28) is movably inserted into the anode high-voltage socket, the insulating plate (28) is configured to cut off or connect a current between the anode high-voltage socket and an external source by moving.

14. The radiotherapy system for atrial fibrillation according to claim 13, wherein the anode high-voltage socket comprises an upper socket (26) and a lower socket (25) fixedly connected via an eccentric shaft (273), wherein the upper socket (26) is fixedly connected to the anode copper bar (29), and there is a channel for the insulating plate (28) to move between the upper socket (26) and the lower socket (25), a combination body (27) is rotatably mounted at the outer end of the eccentric shaft (273), the combination body (27) comprising a conductive portion (271) and an insulating portion (272), the insulating plate (28) and the combination body (27) share a space of the channel, and a side of the insulating portion (272) has a beveled surface for matching with the insulating plate (28).

15. The radiotherapy system for atrial fibrillation according to any one of claims 1 to 9, wherein the arc-shaped X-ray tubes (8) and the arc-shaped X-ray detectors (9) are arranged spaced apart along an axial direction of the ring gantry (7), the X-ray tubes (8) are arranged along an entire circumference within the same cross-section of the ring gantry (7), with a gap (10) for the treatment part (6) to perform treatment, the X-ray detectors (9) are arranged along the entire circumference within the same cross-section of the ring gantry (7), with the gap (10) for the treatment part (6) to perform treatment.
